# EUROPEAN PATENT SPECIFICATION

(11) **EP 1 274 379 B1**
(45) Date of publication and mention of the grant of the patent: **15.03.2006**
(21) Application number: 00954194.7
(22) Date of filing: 16.08.2000
(51) Int. Cl.: A61F 13/15

(54) **THIN SANITARY NAPKIN MANIFESTING HIGH LEVEL OF STABILITY**
DÜNNE DAMENBINDE MIT HOHER STABILITÄT
SERVIETTE HYGIENIQUE FINE PRESENTANT UN NIVEAU DE STABILITE ELEVE

(30) Priority: 16.08.1999 CA 2280389
(43) Date of publication of application: 15.01.2003
(73) Proprietor: JOHNSON & JOHNSON INC., Montreal, Quebec H1N 2G4 (CA); Brisebois, Henri, Lachenaie, Quebec J6W 5W6 (CA); Lariviere, Christiane, Montreal, Quebec H1W 3N4 (CA); Mohmad, Roya, Montreal, Quebec H1M 2W3 (CA)
(72) Inventor: BRISEBOIS, Henri, Lachenaie, Quebec J6W 5W6 (CA); LARIVIERE, Christiane, Montreal, Quebec H1W 3N4 (CA); MOHMAD, Roya, Montreal, Quebec H1M 2W3 (CA)
(74) Representative: Mercer, Christopher Paul
(86) International application number: PCT/CA2000/000935
(87) International publication number: WO 2001/012117

(56) References cited:
- EP-A- 0 305 970
- EP-A- 0 494 112
- EP-A- 0 781 537
- EP-A- 0 804 916
- US-A- 4 518 451
- US-A- 5 779 860
- US-A- 5 866 242

## Description

### FIELD OF THE INVENTION

The present invention relates generally to the art of fluid absorption and more particularly to a disposable sanitary napkin that is thin, highly absorbent and maintains a stable position against the perineal area of the user, both in the dry and in the wet condition.

### BACKGROUND OF THE INVENTION

One element that contributes to the performance of a sanitary napkin is the way the sanitary napkin withstands deformation when in use. It has been observed that the thighs of an individual exert lateral forces on a sanitary napkin when the article is positioned for use on the undergarment. This may results in bunching of the sanitary napkin, affecting the efficiency of the article's ability to collect bodily fluids since there is a concomitant reduction in the article's surface area. This problem is exacerbated when the sanitary napkin has acquired a significant amount of liquid. The liquid mass usually has the effect of reducing the solidity and strength of the structure of the sanitary napkin, which translates in an increased likelihood of bunching.

U.S. patent number 5,866,242 discloses an absorbent material that can be used as an absorbent core in absorbent articles such as feminine hygiene products. The absorbent material contains a blend of cellulosic fibers and a superabsorbent material and can be air-laid as a homogeneous blend of fiber and superabsorbent in one or more layers.

EP 0494112 A1 discloses a highly absorbent and flexible calendered and perf-embossed cellulosic pulp fluff sheet for use in disposable absorbent products such as sanitary napkins and the like. Also disclosed is a method for manufacturing the highly absorbent and flexible pulp fluff sheet and its method of use in disposable absorbent products.

U.S. patent number 4,518,451 discloses a thin absorbent undergarment liner which exhibits a dear, aesthetically pleasing embossed pattern on the body facing surface while still maintaining soft and comfortable longitudinal edges. The liner is provided to have areas of deep depressions on the body facing surface and shallow impressions on the longitudinal edges.

The present invention provides a sanitary napkin adapted to be worn in the crotch portion of an undergarment, the sanitary napkin comprising: a fluid pervious cover layer, an absorbent system under the fuid-previous cover layer, the absorbent system comprising superabsorbent material and cellulosic fibers; a liquid-imprevious barrier layer under the absorbent system; and flape projecting laterally outward from each of two longitudinal sides of the sanitary napkin. The sanitary napkin has a tickness less than about 5 mm. Against this background, it can be seen that there exists a need to provide a sanitary napkin that is highly absorbent and maintains a stable position against the perennial area of the user, both in the dry and in the wet condition.

### SUMMARY OF THE INVENTION

The sanitary napkin manifests an increase in thickness of about 2 times when saturated with liquid. The sanitary napkin has a ratio of lateral stiffness in the saturated condition versus a lateral stiffness in the dry condition of not less than about 0.9. For the purpose of this specification, "saturated condition" refers to a situation where almost all of the capacity of the sanitary napkin has been used up. A "wet condition" refers to a situation where the sanitary napkin contains some liquid but its capacity has not been used up. Accordingly, the sanitary napkin in a wet condition can accept a further insult. It has been observed, that when the ratio of lateral stiffness in the saturated condition versus the dry condition is not less than about 0.9, the sanitary napkin when wet (thus capable of absorbing more liquid), is stable against the perineal area of the wearer and will resist the lateral compression by the thighs of the wearer. As a result, the likelihood of bunching is reduced.

Preferably, the ratio of lateral stiffness in the saturated condition versus the lateral stiffness in the dry condition exceeds 1.

The ability of the sanitary napkin to manifest an increase in thickness of at least about 2 times, when saturated with liquid, provides a sanitary napkin that has a good capacity/size ratio. Overall, the invention provides a sanitary napkin that has a good absorption capacity, is stable when wet, and thus reduce the likelihood of bunching.

Other aspects and features of the present invention will become apparent in those ordinarily skilled in the art upon review of the following description of specific embodiments of the invention in conjunction with the accompanying drawings.

### DESCRIPTION OF THE DRAWINGS

Figure 1 is a top elevational view of a sanitary napkin in accordance with the present invention, the cover layer of the sanitary napkin being partly removed to show the absorbent system;
Figure 2 is a perspective view of the sanitary napkin of Figure 1, depicted in a position attained when the sanitary napkin is placed in the undergarment of a wearer.
Figure 3 is a bottom plan view of the sanitary napkin shown in Figure 1;
Figure 4 is a cross-sectional view taken along the longitudinal axis of the sanitary napkin shown in Figure 3;
Figure 5 is a schematic illustration of means for air-laying absorbent material for making an example of an absorbent layer of the sanitary napkin according to the invention, using four air-laying heads followed by means for compacting the air-laid material;
Figures 6(a) and 6(b) show three and four stratum embodiments, respectively, of the absorbent layer that can be used in the sanitary napkin in accordance with the invention;
Figure 7a depicts an apparatus suitable for performing a lateral compression test on a sanitary napkin, the apparatus being shown without a test sample being placed upon it; and
Figure 7b depicts the apparatus of Figure 7a with a test sample placed upon it and ready for testing.

### DETAILED DESCRIPTION

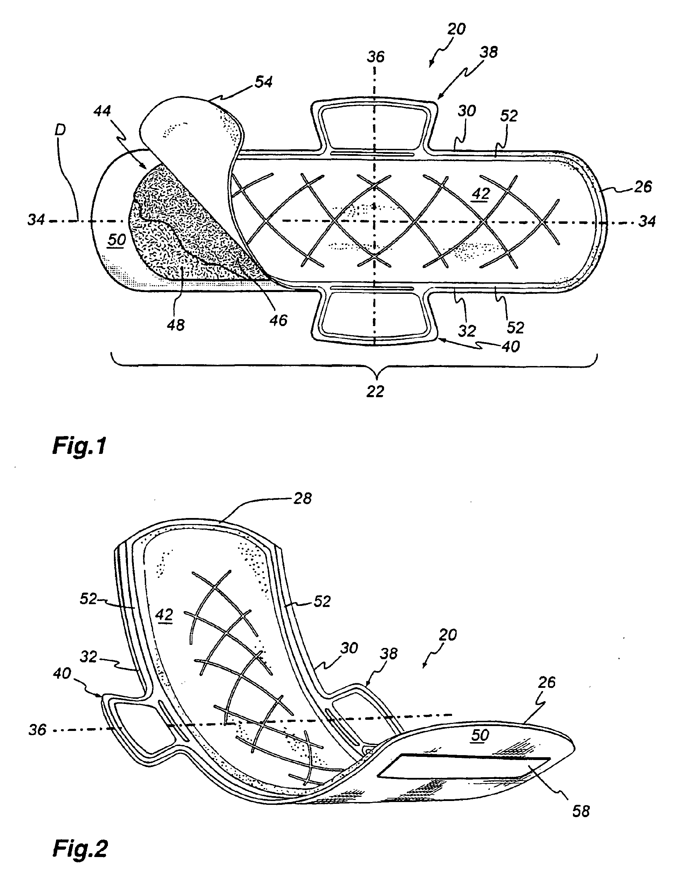

Referring to Figures 1 and 2, there is shown an embodiment of the present invention, a feminine sanitary napkin 20.

The sanitary napkin 20 has a main body 22 with a first transverse side 26 defining a front portion thereof and a second transverse side 28 defining a rear portion thereof. Each of these sides is arcuate or of any other suitable shape. The main body also has two longitudinal sides, namely a longitudinal side 30 and a longitudinal side 32. The sanitary napkin 20 has a thickness not exceeding about 5 mm. Preferably, the thickness is less than 3.5 mm, more preferably less than 3 mm, and most preferably, it is off about 2.8 mm.

The sanitary napkin 20 has a longitudinal centreline 34 that is an imaginary line bisecting the sanitary napkin 20 in two identical halves.

The sanitary napkin 20 shown in the drawings has flaps 38, 40. The flaps 38, 40 projed laterally outward from each of the longitudinal sides 30, 32. The flaps 38, 40 are in the shape of an isosceles trapezoid with the top adjoining the longitudinal side and the base at the distal end. This is an example only as other flap shapes can also be used without departing from the invention.

The main body 22 also has an imaginary transverse centerline 36 perpendicular to the longitudinal centerline 34 and simultaneously bisecting the flaps 38, 40.

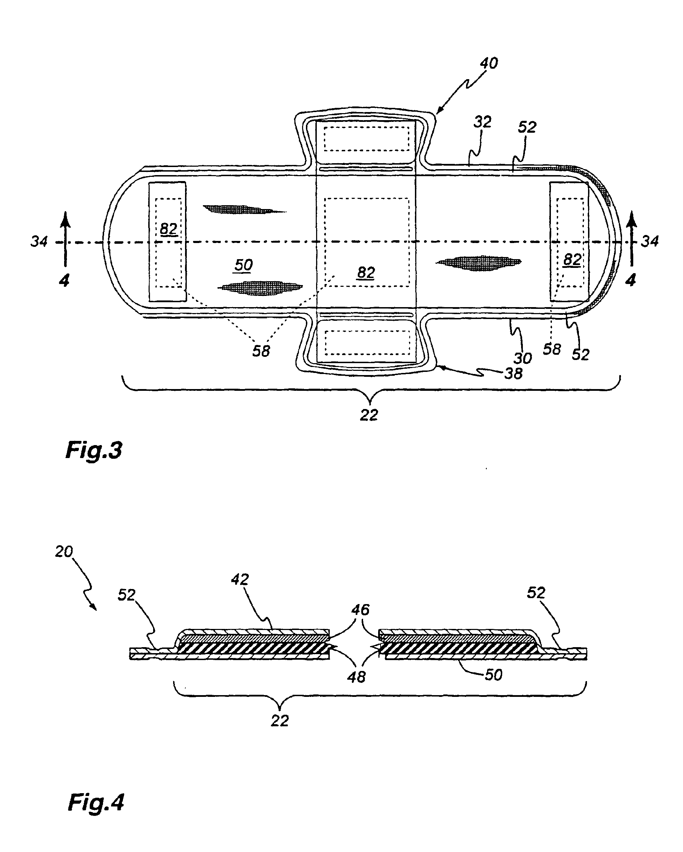

As depicted in Figure 4, the main body 22 is of a laminated construction and preferably comprises a fluid-permeable cover layer 42, an absorbent system 44, and a fluid-impervious barrier layer 50. The absorbent system has preferably two components, namely a first absorbent layer 46 (commonly known as "transfer layer") and a second absorbent layer 48 (commonly known as "absorbent core"). Alternatively, a single layer, namely the second absorbent layer 48, can form the absorbent system 44. Each of these layers is described hereinbelow.

### Main Body-Cover Layer

The cover layer 42 may be a relatively low density, bulky, high-loft non-woven web material. The cover layer 42 may be composed of only one type of fiber, such as polyester or polypropylene or it may be composed of bi-component or conjugate fibers having a low melting point component and a high melting point component. The fibers may be selected from a variety of natural and synthetic materials such as nylon, polyester, polypropylene, rayon (in combination with other fibers), cotton, acrylic fiber and the like and combinations thereof. An example is the non-woven cover layer of sanitary napkins sold by Johnson & Johnson Inc. of Montreal, Canada under the trademark Stayfree Ultra-Thin Cottony Dry Cover.

Bi-component fibers may be made up of a polyester core and a polyethylene sheath. The use of appropriate bi-component materials results in a fusible non-woven fabric. Examples of such fusible fabrics are described in U.S. Patent 4,555,446 issued November 26, 1985 to Mays. Using a fusible fabric increases the ease with which the cover layer may be mounted to the adjacent first absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Advantageously, the fibers which make up the cover layer 42 should not lose their physical properties when they are wetted. In other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polypropylene or bi-component fibers, the cover layer 42 may be treated with a surfactant to impart the desired degree of wettability.

Alternatively, the cover layer 42 can also be made of polymer film having large pores. Because of such high porosity, the film accomplishes the function of quickly transferring body fluid to the inner layers of the absorbent system. Apertured co-extruded films such as described in U.S. Patent 4,690,679 and available on sanitary napkins sold by Johnson & Johnson Inc. of Montreal, Canada could be useful as cover layers in the present invention.

The cover layer 42 may be embossed to the remainder of the absorbent system 44 in order to aid in promoting fluid transport by fusing the cover to the next layer. Such fusion may be effected locally, at a plurality of sites or over the entire contact surface of cover layer 42 with absorbent system 44. Alternatively, the cover layer 42 may be attached to the absorbent system 44 by other means such as by adhesive.

### Main Body-Absorbent System-First Absorbent Laye

Adjacent to the cover layer 42 on its inner side and bonded to the cover layer 42 is a first absorbent layer 46 that forms part of the absorbent system 44. The first absorbent layer 46 provides the means of receiving body fluid from the cover layer 42 and holding it until an underlying second absorbent layer has an opportunity to absorb the fluid.

The first absorbent layer 46 is, preferably, more dense than and has a larger proportion of smaller pores than the cover layer 42. These attributes allow the first absorbent layer 46 to contain body fluid and hold it away from the outer side of the cover layer 42, thereby preventing the fluid from re-wetting the cover layer 42 and its surface. However, the first absorbent layer 46 is, preferably, not so dense as to prevent the passage of the fluid through the layer 46 into the underlying second absorbent layer 48. These types of absorbent layers are commonly known as fluid transfer layers or acquisition layers.

The first absorbent layer 46 may be composed of fibrous materials, such as wood pulp, polyester, rayon, flexible foam, or the like, or combinations thereof. The first absorbent layer 46 may also comprise thermoplastic fibers for the purpose of stabilizing the layer and maintaining its structural integrity. The first absorbent layer 46 may be treated with surfactant on one or both sides in order to increase its wettability, although generally the first absorbent layer 46 is relatively hydrophilic and may not require treatment. The first absorbent layer 46 is preferably bonded on both sides to the adjacent layers, i.e. the cover layer 42 and an underlying second absorbent layer 48. An example of a suitable first absorbent layer is a through air bonded pulp sold by BUCKEYE of Memphis Tennessee under the designation VIZORB 3008.

### Main Body-Absorbent System-Second Absorbent Layer

Immediately adjacent to and bonded to the first absorbent layer 46 is the second absorbent layer 48.

In one embodiment, the first absorbent layer 46 has a central width that is at least about the same as the central width of the second absorbent layer 48. In a specific embodiment, this central width is greater than about 64mm. In another embodiment, the first absorbent layer 46 has a central width that exceeds the central width of the second absorbent layer 48. The term "central width" refers to a specific area of a layer, such as an absorbent layer determinable as follows. A reference point on the sample layer that is disposed beneath the center of the vaginal orifice, when worn, is located. A plane parallel to the transverse centerline 36 and 3.75 centimeters forward from the reference point in the direction of the wearer's mons pubis is located. Another plane parallel to the lateral centerline 36 and 5.0 centimeters rearward from the reference point in the direction of the wearer's buttocks is also located. The greatest flat-out, uncompressed, unmanipulated, lateral width of the sample layer between the two planes is the absorbent width of the sample layer.

The central width of the absorbent system, when the absorbent system includes a plurality of absorbent layers is the central width of the layer of the absorbent system that has the largest central width. In a specific example, the central width of the absorbent system exceeds 64 mm.

In one embodiment, the second absorbent layer 48 is a blend or mixture of cellulosic fibers and superabsorbent disposed in and amongst fibers of that pulp.

In a specific example, the second absorbent layer 48 is a material containing from about 40 weight percent to about 95 weight percent cellulosic fibers; and from about 5 weight percent to about 60 weight percent SAP (superabsorbent polymers). The material has a water content of less than about 10 weight percent. As used herein, the phrase "weight percent" means weight of substance per weight of final material. By way of example, 10 weight percent SAP means 10 g/m2 SAP per 100g/m2 basis weight of the material.

Cellulosic fibers that can be used in the second absorbent layer 48 are well known in the art and include wood pulp, cotton, flax and peat moss. Wood pulp is preferred. Pulps can be obtained from mechanical or chemi-mechanical, sulfite, Kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material.

The second absorbent layer 48 can contain any superabsorbent polymer (SAP), which SAPs are well known in the art. For the purposes of the present invention, the term "superabsorbent polymer" (or "SAP") refers to materials which are capable of absorbing and retaining at least about 10 times their weight in body fluids under a 0.5 psig pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA60N Type II*, and the product offered by Chemdal International, Inc. of Palatine, Illinois, under the designation of 2100A*.

In a specific example the second absorbent layer 48 is a material containing from about 50 to about 95 weight percent cellulosic fibers and, more specifically from about 60 to about 80 weight percent cellulosic fibers. Such a material may contain from about 5 to about 60 weight percent SAP, preferably from about 20 to about 55 weight percent SAP, even more preferably from about 30 to about 45 weight percent SAP, and most preferably about 40 weight percent SAP.

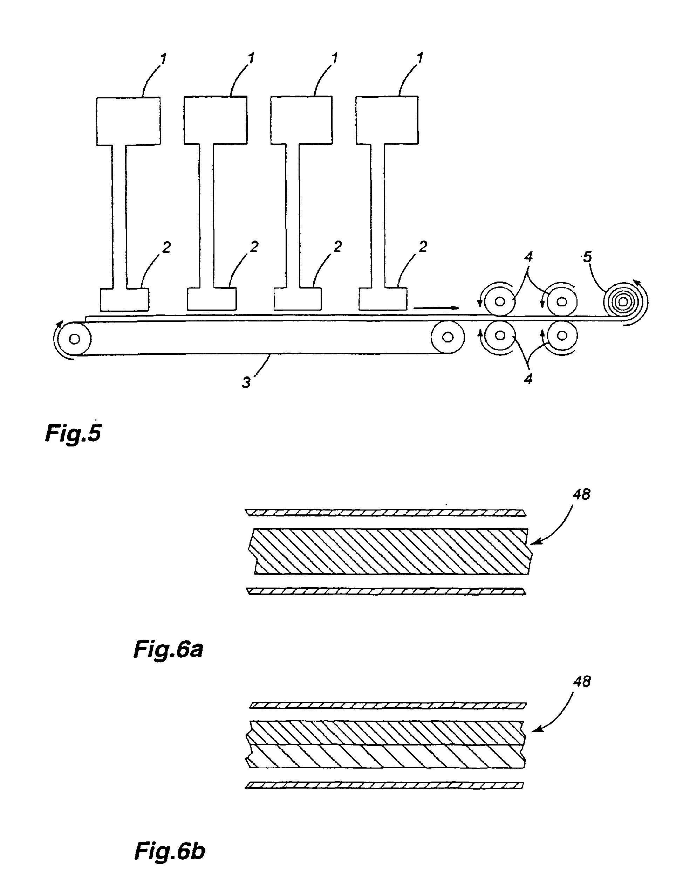

The second absorbent layer 48 can be manufactured by using air-laying means well known in the art (See Figure 5). In accordance with Figure 5, cellulosic fibers (e.g., pulp) are processed using a hammer mill to individualize the fibers. The individualized fibers are blended with SAP granules in a blending system 1 and pneumatically conveyed into a series of forming heads 2. The blending and distribution of fibers and SAP granules can be controlled separately for each forming head. Controlled air circulation and winged agitators in each chamber produce uniform mixture and distribution of pulp and SAP. The SAP can be thoroughly and homogeneously blended throughout the material or contained only in specific strata by distributing it to selected forming heads. Fibers (and SAP) from each forming chamber are deposited by vacuum onto a forming wire 3 thus forming a layered absorbent web. The web is subsequently compressed using calenders 4 to achieve desirable density. The densified web is wound into a roll 5 using conventional winding equipment. The forming wire 3 can be covered with tissue paper to reduce the loss of material. The tissue paper layer can be removed prior to calendering or incorporated into the formed material. In a possible variant, the first absorbent layer 46 can be formed integrally with the second absorbent layer 48 to provide a unitized absorbent system 44. This can be achieved by providing the apparatus depicted in Figure 5 with an additional forming head (not shown in the drawings) to deposit on the second absorbent layer 48, by air laying and prior to calendering, a layer of material to form the first absorbent layer 46.

The second absorbent layer 48 of the present invention is of high density and in a specific example has a density of greater than about 0.25 g/cc. Specifically, the second absorbent layer 48 may have a density in the range of from about 0.30 g/cc to about 0.50 g/cc. More specifically, the density is from about 0.30 g/cc to about 0.45 g/cc and, even more specifically from about 0.35 g/cc to about 0.40 g/cc.

Air-laid absorbents are typically produced with a low density. To achieve higher density levels, such as the examples of the second absorbent layer 48 given above, the air-laid material is compacted using calenders as shown in Figure 5. Compaction is accomplished using means well known in the art. Typically such compaction is carried out at a temperature of about 100 degrees C and a load of about 130 Newtons per millimeter. The upper compaction roll is typically made of steel while the lower compaction roll is a flexroll having a hardness of about 85 SH D. It is preferred that both the upper and lower compaction rolls be smooth, although the upper roll can be engraved.

In one embodiment the second absorbent layer 48 has a ratio of Gurley stiffness, measured in milligrams (mg) to density, measured in grams per cubic centimeter (g/cc), of less than about 3700. In a specific example, that ratio of Gurley stiffness to density is less than about 3200 and, more specifically, less than about 3000.

Gurley stiffness is one of many indices of softness. Gurley stiffness measures the bendability or flexibility of absorbent materials. The lower the Gurley stiffness value, the more flexible the material. The Gurley stiffness values are measured using a Gurley Stiffness Tester (Model No. 4171E), manufactured by Gurley Precision Instruments of Troy, N.Y. The instrument measures the externally applied moment required to produce a given deflection of a test strip of specific dimensions fixed at one end and having a concentrated load applied to the other end. The results are obtained in "Gurley Stiffness" values in units of milligrams.

The second absorbent layer 48 is strong in light of its softness. Pad integrity is a well-known measurement of absorbent material strength. In a specific embodiment the second absorbent layer 48 demonstrates strength (high pad integrity) over a wide range of densities. In a specific example the second absorbent layer 48 has a pad integrity, measured in Newtons (N), to density (g/cc) ratio of greater than about 25.0. In a more specific example, that ratio is greater than about 30.0 and, could even be greater than about 35.0. The pad integrity is a test performed on an Instron Universal Testing Machine. Essentially, the test measures the load required to pierce through the test sample, as described in the PFI Method of 1981. A test sample having dimensions of 50 mm by 50 mm is clamped on the Instron with a suitable fastening device. A 20 mm diameter piston traveling at the rate of 50 mm/min punctures the stationary sample. The force required to puncture the sample is measured in Newtons (N).

The second absorbent layer 48 can be prepared over a wide range of basis weights. The second absorbent layer 48 can have a basis weight in the range of from about 100 g/m2 to about 700 g/m2. In a specific example, the basis weight ranges from about 150 g/m2 to about 350 g/m2. Preferably the basis weight ranges from about 200 g/m2 to about 300 g/m2 and, more preferably, to about 250 g/m2.

The second absorbent layer 48 can be formed as three or four lamina or strata. Those strata include a bottom layer, one or two middle layers and a top layer. Specific examples of three and four layer material are set forth below. The SAP can be included in any or all of the layers. The concentration (weight percent) of SAP in each layer can vary as can the nature of the particular SAP.

An interesting characteristic of the second absorbent layer 48 is its ability to retain SAP when subjected to mechanical stress. The second absorbent layer 48 retained over 85 percent by weight of its SAP content when subjected to 10 minutes of rigorous shaking. Specifically, a material of this invention retains over 90 percent, more specifically over 95 percent and, even more specifically over 99 percent of its SAP under these mechanical stresses. The percent of SAP retained was determined by shaking the material in a Ro-Tap Sieve Shaker manufactured by W. S. Tyler Co., Cleveland Ohio. More specifically the sample is placed in a 28-mesh (Tyler series) sieve. Additional sieves of 35-mesh and 150-mesh were attached to the first sieve forming a column of increasingly fine sieves. The column of sieves was capped on either end to prevent the loss of fiber and/or SAP. The sieve column was placed in the shaker and agitated for 10 minutes. The amount of SAP granules shaken loose from the sample, "free SAP", was determined by combining the residue contained in each of the sieves and separating the cellulosic fiber from the SAP.

Even where prepared as from multiple layers, the final thickness of the formed second absorbent layer 48 is low. The thickness can vary from about 0.5 mm to about 2.5 mm. In a specific example, the thickness is from about 1.0 mm to about 2.0 mm and, even more specifically from about 1.25 mm to about 1.75 mm.

One embodiment of the second absorbent layer 48 particularly well suited for use in the sanitary napkin 20 is depicted in Figure 6. Such second absorbent layer 48 has a basis weight of from about 200 g/m2 to about 350 g/m2 and a density between about 0.3 g/cc and 0.5 g/cc. In a specific example, the density is from about 0.3 g/cc to about 0.45 g/cc and, more specifically about 0.4 g/cc.

The second absorbent layer 48 depicted in Figure 6(a) is air-laid as three strata: a bottom layer of pulp (without superabsorbent) with a basis weight of about 25 g/m2; a middle layer with a basis weight of about 150 g/m2 and which contains from about 10 to about 30 g/m2 superabsorbent and from about 120 g/m2 to about 140 g m2 pulp; and a top layer of pulp (without superabsorbent) with a basis weight of about 25 g/m2. Relative to the total basis weight of the second absorbent layer 48, the level of superabsorbent ranges from about 5 to about 15 weight percent (g/m2 of superabsorbent per g/m2 material). In a specific example, the level of superabsorbent is from about 7.5 weight percent to about 12.5 weight percent of the material. More specifically, the material contains about 10 weight percent of superabsorbent. Thus, the middle layer of the material could contain from about 15 g/m2 to about 25 g/m2 superabsorbent and from about 125 g/m2 to about 135 g/m2 pulp and, more specifically about 20 g/m2 superabsorbent and about 130 g/m2 pulp. The middle layer containing pulp and superabsorbent can be laid down as a homogeneous blend or as a heterogeneous blend wherein the level of superabsorbent varies with proximity to the bottom layer.

In another embodiment depicted in Figure 6(b), the second absorbent layer 48 is air-laid as four strata. In this embodiment, the middle layer referred to above is replaced with two middle layers: a first middle layer adjacent the top layer and a second middle layer adjacent the bottom layer. Each of the first and second middle layers independently comprises from about 10 to about 30 g/m2 superabsorbent and from about 40 g/m2 to about 65 g/m2 pulp. When it is desired to keep absorbed fluid away from the cover layer 42 the amount of superabsorbent in the first and second middle layers is adjusted such that there is a higher level of superabsorbent in the second middle layer. The superabsorbent in the first and second middle layers can be the same or a different superabsorbent.

In one embodiment, the cellulosic fiber for use in the second absorbent layer 48 is wood pulp. There are certain characteristics of wood pulp that make it particularly suitable for use. Cellulose in most wood pulps has a crystalline form known as Cellulose I which can be converted to a form known as Cellulose II. In the second absorbent layer 48, wood pulp with a substantial portion of the cellulose as Cellulose II could be used. Similarly, pulps having an increased fiber curl value are advantageous. Finally, pulps having reduced levels of hemicellulose are preferred. Means for treating pulps so as to optimize these characteristics are well known in the art. By way of example, treating wood pulp with liquid ammonia is known to convert cellulose to the Cellulose II structure and to increase the fiber curl value. Flash drying is known to increase the fiber curl value of pulp. Cold caustic treatment of pulp decreases hemicellulose content, increases fiber curl and converts cellulose to the Cellulose II form. Thus it could be advantageous that the cellulosic fibers used to produce the material of this invention contain at least a portion of cold caustic treated pulp.

A description of the cold caustic extraction process can be found in U.S. patent application Ser. No. 08/370,571, filed on Jan. 18, 1995, pending which application is a continuation-in-part application of U.S. patent application Ser. No. 08/184,377, now abandoned filed on Jan. 21, 1994.

Briefly, a caustic treatment is typically carried out at a temperature less than about 60 degree C., but preferably at a temperature less than 50 degree C., and more preferably at a temperature between about 10 degree C. to 40 degree C. A preferred alkali metal salt solution is a sodium hydroxide solution newly made up or as a solution by-product in a pulp or paper mill operation, e.g., hemicaustic white liquor, oxidized white liquor and the like. Other alkali metal salts such as ammonium hydroxide and potassium hydroxide and the like can be employed. However, from a cost standpoint, the preferable salt is sodium hydroxide. The concentration of alkali metal salts is typically in a range from about 2 to about 25 weight percent of the solution, and preferably from about 6 to about 18 weight percent. Pulps for high rate, fast absorbing applications are preferably treated with alkali metal salt concentrations from about 10 to about 18 weight percent.

For further details on the structure and the method of construction of the second absorbent layer 48 the reader is invited to refer to the US patent 5,866,242 granted on February 2, 1999 to Tan et al.

### Main Body-Barrier Layer

Underlying the absorbent system 44 is a barrier layer 50 comprising liquid-impervious film material so as to prevent liquid that is entrapped in the absorbent system 44 from egressing the sanitary napkin and staining the wearer's undergarment. The barrier layer 50 is made preferably of polymeric film.

The cover layer 42 and the barrier layer 50 are joined along their marginal portions so as to form an enclosure or flange seal that maintains the absorbent system 44 captive. The joint may be made by means of adhesives, heat-bonding, ultrasonic bonding, radio frequency sealing, mechanical crimping, and the like and combinations thereof. The peripheral seal line is shown in Figure 1 by the reference numeral 52.

### Flaps

The flaps 38 and 40 are preferably made as integral extensions of the cover layer 42 and the barrier layer 50. These integral extensions are joined to one another along their marginal seal portions by adhesives, heat-bonding, ultrasonic bonding, radio frequency sealing, mechanical crimping, and the like and combinations thereof. Most preferably, such joining is made at the same time the cover layer 42 and the barrier layer 50 are bonded to one another to enclose the absorbent system 44. Alternatively, the flaps may include absorbent material between the cover layer and the barrier layer extensions. Such absorbent material may be an extension of the first absorbent layer 46, the second absorbent layer 48 or both. The flaps may have another suitable shape than the ones shown.

### Adhesive system

Referring to Figures 2 and 3, in order to enhance the stability of the sanitary napkin, the garment facing surface of the barrier layer is provided with positioning adhesive material 58, typically hot-melt adhesive material capable of establishing a temporary bond with the undergarment material. A suitable material is the composition designated HL-1491 XZP commercially available from H.B. Fuller Canada, Toronto, Ontario, Canada. The positioning adhesive 58 may be applied to the garment-facing surface of the barrier layer 50 in various patterns, including complete adhesive coverage, parallel longitudinal lines, a line of adhesive following the perimeter of the structure, transverse lines of adhesive or the like.

Standard release paper 82 (shown only in Figure 3) covers the positioning adhesive 58 before the napkin is used to prevent the unwanted adherence of the napkin to itsetf or foreign objects. The release paper is of conventional construction (e.g. silicone coated wet-laid Kraft wood pulp) and sukable papers are wailable from Tekkote Corporation (Leonia, New Jersey, USA), and bear the designation FRASER 30#/61629.

### Method of manufacture

The above-described embodiment of the sanitary napkin 20 is fabricated in a conventional manner in accordance with conventional techniques. Specifically, a laminate structure, sometimes referred to in the art as a web, is created. This laminate structure comprises an expanse of the materials from which the napkin will be created. i.e. the laminate structure comprises the following layers of material in a top-to-bottom order, an expanse of cover layer material; an expanse of first absorbent layer material; an expanse of second absorbent layer material (manufactured as described above); and finally an expanse of barrier layer. Some of the materials are necessarily not continuous within the laminate structure, and where such is the case, they are positioned precisely, one with respect to another, in the relationship they will occupy in the final products. The cover layer material and the barrier layer material are then bonded together by applying pressure in the appropriate positions, and what will become the peripheral seal is created. (The seal may also be made by means of heat-bonding, ultrasonic bonding, radio frequency sealing, mechanical crimping, and the like and combinations thereof.) The sealed structure is then severed by conventional means (i.e. die-cutting, fluid-jet cutting, or by laser) from the web to create a discrete article.

The positioning adhesive material is then applied to the barrier layer in the appropriate positions, and release paper is applied to cover the positioning adhesive. Alternatively, the positioning adhesive, or the positioning adhesive and the release paper may be applied to the web before the individual articles are severed therefrom.

As indicated earlier, the sanitary napkin 20 has a thickness of about 5 mm or less in the dry state. The apparatus required to measure the thickness of the sanitary napkin is a footed dial (thickness) gauge, available from Ames, with a 2" diameter foot and readout accurate to 0.001". A digital type apparatus is preferred.

If the sanitary napkin sample is individually folded and wrapped, the sample is unwrapped and carefully flattened by hand. The release paper is removed from the sample and it is repositioned back gently across the positioning adhesive lines so as not to compress the sample, ensuring that the release paper lies flat across the sample.

The foot of the gauge is raised and the sample is placed on the anvil such that the foot of the gauge is approximately centered to the sample (or in the location of interest on the sample of interest). When lowering the foot, care is taken to avoid allowing the foot to "drop" or that undue force is applied. A load of 0.07 p.s.i.g. is applied to the sample and the readout is allowed to stabilise for approximately 5 seconds. The thickness reading is then taken. The thickness of the release paper covering the positioning adhesive is deducted from the total thickness.

The same procedure is followed when the thickness of the sanitary napkin is measured in the saturated condition.

The sanitary napkin 20 is characterised by excellent absorption properties and at the same time it has a level of lateral stiffness sufficient to reduce the incidence of bunching in use, particularly when the sanitary napkin is in the wet condition. More particularly, the sanitary napkin 20 manifests an increase in thickness of at least about 2 times when saturated with liquid. The increase in thickness is measured as follows. First the thickness of the sanitary napkin in the dry condition is measured by the test detailed above. A sample in the dry condition is a sample to which no liquid has been added and that has been conditioned at 50% relative humidity in a room at 21 degrees C for two hours. The dry thickness value is recorded. The sanitary napkin is then saturated with liquid. This is performed as follows: Using a 25 ml graduated cylinder, 15 ml of 1% Saline Solution is added to the napkin through a plastic template that has an oval opening. The template is removed and after a waiting period of 5 minutes, the thickness is performed as per the test method described above. The thickness increase is expressed as the ratio of wet dry thickness.

The sanitary napkin has a ratio of lateral stiffness (after cycle 1) in the saturated condition versus the dry condition of not less that about 0.9 and preferably exceeding 1. The lateral stiffness is measured using the Curved Longitudinal Bending Test simulates the lateral compressive forces exerted on sanitary napkins in actual use.

The equipment and tests as performed are as follows:

### a) Equipment and Material

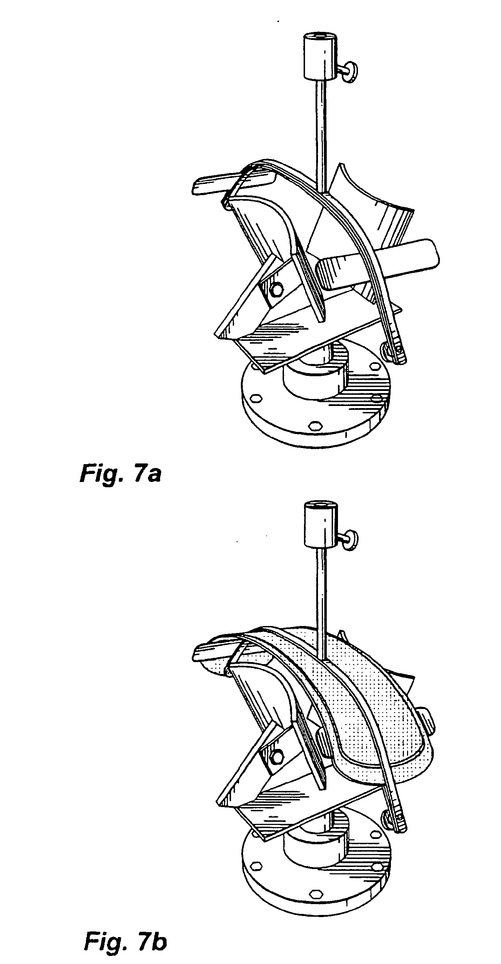

- SINTECH TESTWORKS FOR WINDOWS® by MTS® with Instron Load Frame. This software runs on a suitable computer. It runs, displays and records statistical analysis on measurements obtained while running the Lateral Compressive Test Unit as described below.
- Curved Longitudinal Bending Test Set-up as depicted in Figures 7a and 7b. This is composed of:
   - Two (2) smooth curved (convexed) stainless steel plates at an angle of 40°. The distance between the plates at the top is 73 mm and the bottom is 18 mm. The radius of the arc is 68 mm and the segment length is 113 mm for the convexed plates. The bottom portion is concave in the vertical direction. The radius of the arc is 108 mm and the segment length is 113 mm. These plates are attached onto a jig. This set-up represents the crotch and leg area of the female anatomy.
   - A curved holder (clamp type) having an overall length of 280 mm and a width of 6 mm, is connected to a support shaft of 156 mm x 6.35 mm (diameter). The shaft is connected to an adapter. The adapter is attached to a tensiometer cell. This unit measures the force necessary to compress the sample to a specified width. In this test procedure the distance is 18 mm.

### b) Sample Preparation

Once the samples have been measured for thickness (wet or dry) the release papers are removed. The tabs (if applicable) are folded at the junction points and attached to the positioning adhesive on the backside of the napkin. A small amount of talcum powder is brushed onto the remaining exposed adhesive.

### c) Procedure

The prepared napkin is placed in the center of the curved clamp with the backside facing the shaft and fastened into place, as shown in Figure 7b.

The movable curved clamp unit is then allowed to cycle 5 times up and down [at a speed of 50 cm/minute] compressing the napkin width down to 18 mm each time. In the first cycle the napkin travels down a total of 85 mm and stops. The first reading is registered. It then returns to the 75 mm mark, stops and then returns to the 85 mm mark and stops. The 2^{nd} reading is registered. The 3^{rd}, 4^{th}, 5^{th} cycles are done in the same fashion as the 2^{nd}.

Typical average values for both the Thickness and Lateral Stiffness can be found in the following table:

**TABLE OF LATERAL STIFFNESS AND THICKNESS**

| **PRODUCT IDENTIFICATION 1999** | **CURVED LONGITUDINAL BENDING grams -[DRY]** | | **CURVED LONGITUDINAL BENDING grams -[WET]** | | **RATIO OF WET/DRY** | | **THICKNESS @0.1 p.s.i.g.** | | |
|---|---|---|---|---|---|---|---|---|---|
| | Cycle 1 | Cycle 5 | Cycle | Cycle 5 | C1/C1 | C5/C1 | **Dry** (mm) | **Wet** [mm] | **Ratio** D/W |
| always UT Maxi with Flexi-Wings | 244 | 110 | 121 | 68 | 0,50 | 0.28 | 2.49 | 2.97 | 1.19 |
| Kotex® UT Maxi with Wings | 621 | 476 | 368 | 275 | 0.59 | 0.44 | 3.94 | 3.99 | 1.01 |
| Nana® Invisible Clip | 648 | 458 | 200 | 122 | 0.31 | 0.19 | 2.59 | 4.30 | 1.66 |
| Metro UT Maxi with Flaps | 408 | 212 | 298 | 106 | 0.73 | 0.26 | 3.21 | 5.03 | 1.57 |
| Incognito UT Maxi with Adjust Tabs® | 506 | 291 | 354 | 219 | 0.70 | 0.43 | 2.79 | 5.82 | 2.09 |
| Staytree* UT Maxi with Wings | 392 | 304 | 301 | 220 | 0.77 | 0.56 | 2.63 | 4.00 | 1.52 |
| Prototype A with Wings | 187 | 128 | 266 | 119 | 1.42 | 0.64 | 2.41 | 5.03 | 2.09 |
| Prototype B with Wings | 238 | 160 | 258 | 90 | 1.08 | 0.38 | 1.80 | 4.55 | 2.53 |
| Prototype C with Wings | 182 | 118 | 242 | S9 | 1.33 | 0.49 | 2.29 | 5.06 | 2.21 |
| Prototype D with Wings | 244 | 170 | 231 | 78 | 0.95 | 0.31 | 1.60 | 3.66 | 2.29 |

A list of typical components used in making prototypes are found in the following table:

**TYPICAL COMPONENTS USED IN PROTOTYPES**

| **PRODUCT IDENTIFICATION** | **COVER TYPE** | **TRANFER LAYER** | **ABSORBENT CORE** |
|---|---|---|---|
| **Prototype A** | 34 gsm Nonwoven [PGI] | 90 gsm WNN [Fort James] | 250 gsm [40% SAP] (Rayonier) |
| **Prototype B** | 34 gsm Nonwoven [PGI] | 65 gsm [Fort James] | 250 gsm [40% SAP] (Rayonier) |
| **Prototype C** | 34 gsm Nonwoven [PGI] | 90 gsm X-353 (Buckeye) | 250 gsm [40% SAP] (Rayonier) |
| **Prototype D** | 34 gsm Nonwoven [PGI] | 65 gsm [Fort James] | 200 gsm [30% SAP](Rayonier) |

Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims.

## Claims

1. A sanitary napkin (20) adapted to be worn in the crotch portion of an undergarment, said sanitary napkin (20) comprising:
a fluid pervious cover layer (42);
an absorbent system (44) under said fluid-pervious cover layer (42), said absorbent system comprising superabsorbent material and cellulosic fibers; and
a liquid-impervious barrier layer (50) under said absorbent system (44),
wherein:
said sanitary napkin (20) has a first lateral stiffness in a saturated condition; and
said sanitary napkin (20) has a second lateral stiffness in a dry condition;
**characterized in that**
said sanitary napkin (20) has a thickness less than about 5 mm;
said sanitary napkin (20) manifests an increase in thickness of at least about 2 times when saturated with liquid;
a ratio of said first lateral stiffness to said second lateral stiffness is not less than about 0.9; and
the sanitary napkin (20) further comprises flaps (38, 40) projecting laterally outward from each of two longitudinal sides (30, 32) of said sanitary napkin (20).

2. A sanitary napkin as defined in claim 1, wherein the ratio of the lateral stiffness in the saturated condition versus the lateral stifmess in the dry condition exceeds 1.

3. A sanitary napkin as defined in claim 1 or claim 2, wherein the thickness of the sanitary napkin in the dry condition is less than about 3 mm and is preferably about 2.8 mm.

4. A sanitary napkin as defined in any one of claims 1, to 3, wherein said absorbent system includes a blend of cellulosic fibers and superabsorbent material.

5. A sanitary napkin as defined in claim 4, wherein said absorbent system comprises an absorbent layer (48) having a basis weight of from 100 g/m2 to 700 g/m2 which has been air-laid as a bottom stratum of pulp, a middle stratum of pulp and superabsorbent polymer disposed in amongst the pulp, and a top stratum containing at least some pulp.

6. A sanitary napkin as defined in claim 5, wherein said absorbent layer has a density of more than about 025 g/cc, preferably in the range from 0.3 g/cc to 0.5 g/cc, more preferably in the range from 0.3 g/cc to 0.45 g/cc.

7. A sanitary napkin as defined in claim 5 or claim 6, wherein said absorbent layer includes from 5 weight percent to 60 weight percent, preferably from 20 weight percent to 55 weight percent, more preferably from 30 weight percent to 45 weight percent, and most preferably about 40 weight percent, superabsorbent polymer.

8. A sanitary napkin as defined in claim 4 or any claim dependent thereon, wherein said absorbent layer is a second absorbent layer (48) and said absorbing system further comprising a first absorbent layer (46) above said second absorbent layer (48).

9. A sanitary napkin as defined in claim 8, wherein said first absorbent layer is air laid over the top stratum of pulp of said second layer.

10. A sanitary napkin as defined in any one of claims 1 to 9, wherein said sanitary napkin includes a fastener (58) for retaining said sanitary napkin on an undergarment of a wearer.

11. A sanitary napkin as defined in claim 8 or any claim dependent thereon, wherein said second absorbent layer has a basis weight in the range from 150 g/m² to 350 g/m², preferably from 200 g/m² to 300 g/m², more preferably about 250 g/m².

12. A sanitary napkin as defined in claim 5 or any claim dependent thereon, wherein the middle stratum comprises a first middle stratum adjacent the bottom stratum and a second middle stratum adjacent the top stratum.

## Patentansprüche

1. Damenbinde (20), die geeignet ist, im Genitalbereich einer Unterkleidung getragen zu werden, wobei die Damenbinde (20) umfaßt:
eine flüssigkeitsdurchlässige Abdecklage (42);
ein absorbierendes System (44) unter der flüssigkeitsdurchlässigen Abdecklage (42), wobei das absorbierende System superabsorbierendes Material und Zellstoffasern umfaßt; und
eine flüssigkeitsundurchlässige Absperrlage (50) unter dem absorbierenden System (44), wobei:
die Damenbinde (20) eine erste seitliche Steifigkeit im gesättigten Zustand aufweist; und
die Damenbinde (20) eine zweite seitliche Steifigkeit im trockenen Zustand aufweist, **dadurch gekennzeichnet, daß**:
die Damenbinde (20) eine Dicke von weniger als zirka 5 mm aufweist;
die Damenbinde (20) eine zumindest zirka zweifache Zunahme der Dicke zeigt, wenn sie mit Flüssigkeit gesättigt ist;
das Verhältnis der ersten seitlichen Steifigkeit zur zweiten seitlichen Steifigkeit nicht geringer als zirka 0,9 ist; und
die Damenbinde (20) weiterhin Klappen (38, 40) umfaßt, die sich seitlich nach außen von jeder der zwei longitudinalen Seiten (30, 32) der Damenbinde (20) erstrecken.

2. Damenbinde, wie in Anspruch 1 definiert, **dadurch gekennzeichnet, daß** das Verhältnis der seitlichen Steifigkeit im gesättigten Zustand zur seitlichen Steifigkeit im trockenen Zustand größer als 1 ist.

3. Damenbinde, wie in Anspruch 1 oder Anspruch 2 definiert, **dadurch gekennzeichnet, daß** die Dicke der Damenbinde im trockenen Zustand kleiner als zirka 3 mm und vorzugsweise zirka 2,8 mm ist.

4. Damenbinde, wie in einem der Ansprüche 1 bis 3 definiert, **dadurch gekennzeichnet, daß** das absorbierende System eine Mischung von Zellstoffasern und superabsorbierendem Material einschließt.

5. Damenbinde, wie in Anspruch 4 definiert, **dadurch gekennzeichnet, daß** das absorbierende System eine absorbierende Lage (48) mit einem Basisgewicht von 100 g/m² bis 700 g/m² umfaßt, welche luftgelegt worden ist als eine Grundschicht aus Zellstoff, eine Mittelschicht aus Zellstoff und superabsorbierendem Polymer, das innerhalb des Zellstoffs angeordnet ist, und eine oberste Schicht, die zumindest etwas Zellstoff enthält.

6. Damenbinde, wie in Anspruch 5 definiert, **dadurch gekennzeichnet, daß** die absorbierende Lage eine Dichte von mehr als zirka 0,25 g/cc hat, vorzugsweise im Bereich von 0,3 g/cc bis 0,5 g/cc, und besser im Bereich von 0,3 g/cc bis 0,45 g/cc.

7. Damenbinde, wie in Anspruch 5 oder Anspruch 6 definiert, **dadurch gekennzeichnet, daß** die absorbierende Lage superabsorbierendes Polymer mit einem Massenanteil von 6 bis 60, vorzugsweise von 20 bis 55, noch besser von 30 bis 45 und am besten von zirka 40 einschließt.

8. Damenbinde, wie in Anspruch 4 oder in einem von diesem abhängigen Anspruch definiert, **dadurch gekennzeichnet, daß** die absorbierende Lage eine zweite absorbierende Lage (48) darstellt und daß das absorbierende System weiterhin eine erste absorbierende Lage (46) über der zweiten absorbierenden Lage (48) umfaßt.

9. Damenbinde, wie in Anspruch 8 definiert, **dadurch gekennzeichnet, daß** die erste absorbierende Lage über der obersten Schicht Zellstoff der zweiten Lage luftgelegt ist.

10. Damenbinde, wie in einem der Ansprüche 1 bis 9 definiert, **dadurch gekennzeichnet, daß** die Damenbinde eine Befestigung (58) für das Zurückhalten der Damenbinde auf einer Unterkleidung einer Trägerin enthält.

11. Damenbinde, wie in Anspruch 8 oder in einem von diesem abhängigen Anspruch definiert, **dadurch gekennzeichnet, daß** die zweite absorbierende Lage ein Basisgewicht im Bereich von 150 g/m² bis 350 g/m², vorzugsweise von 200 g/m² bis 300 g/m² und besser von zirka 250 g/m² aufweist.

12. Damenbinde, wie in Anspruch 5 oder in einem von diesem abhängigen Anspruch definiert, **dadurch gekennzeichnet, daß** die mittlere Schicht eine erste mittlere Schicht angrenzend an die untere Schicht und eine zweite mittlere Schicht angrenzend an die oberste Schicht umfaßt.

## Revendications

1. Serviette hygiénique (20) adaptée pour être portée dans la partie d'entrejambe d'un sous-vêtement, ladite serviette hygiénique (20) comportant :
une couche de recouvrement perméable à un fluide (42),
un système absorbant (44) sous ladite couche de recouvrement perméable à un fluide (42), ledit système absorbant comportant un matériau super-absorbant et des fibres cellulosiques, et
une couche barrière imperméable à un liquide (50) sous ledit système absorbant (44), où :
ladite serviette hygiénique (20) a une première rigidité latérale dans un état saturé, et
ladite serviette hygiénique (22) a une seconde rigidité latérale dans un état sec,
**caractérisée en ce que** :
ladite serviette hygiénique (20) a une épaisseur inférieure à environ 5 mm,
ladite serviette hygiénique (20) manifeste une augmentation d'épaisseur qui est multipliée au moins environ par deux lorsqu'elle est saturée d'un liquide,
un rapport de ladite première rigidité latérale sur ladite seconde rigidité latérale n'est pas inférieur à environ 0,9, et
la serviette hygiénique (20) comporte en outre des volets (38, 40) faisant saillie latéralement vers l'extérieur à partir de chacun de deux côtés longitudinaux (30, 32) de ladite serviette hygiénique (20).

2. Serviette hygiénique selon la revendication 1, dans laquelle le rapport de la rigidité latérale dans l'état saturé sur la rigidité latérale dans l'état sec dépasse 1.

3. Serviette hygiénique selon la revendication 1 ou 2, dans laquelle l'épaisseur de la serviette hygiénique dans l'état sec est inférieure à environ 3 mm, et est de préférence d'environ 2,8 mm.

4. Serviette hygiénique selon l'une quelconque des revendications 1 à 3, dans laquelle ledit système absorbant comporte un mélange de fibres cellulosiques et d'un matériau super-absorbant.

5. Serviette hygiénique selon la revendication 4, dans laquelle ledit système absorbant comporte une couche absorbante (48) ayant un grammage de 100 g/m² à 700 g/m² qui a été formé par air-laid sous la forme d'une strate de pulpe inférieure, d'une strate de pulpe médiane et d'un polymère super-absorbant disposé parmi la pulpe, et d'une strate supérieure contenant au moins une certaine quantité de pulpe.

6. Serviette hygiénique selon la revendication 5, dans laquelle ladite couche absorbante a une densité supérieure à environ 0,25 g/cc, de préférence dans la plage de 0,3 g/cc à 0,5 g/cc, de manière plus préférée dans la plage de 0,3 g/cc à 0,45 g/cc.

7. Serviette hygiénique selon la revendication 5 ou 6, dans laquelle ladite couche absorbante comporte de 5 % en poids à 60 % en poids, de manière préférée de 20 % en poids à 55 % en poids, de manière plus préférée de 30 % en poids à 45 % en poids, et de la manière la plus préférée d'environ 40 % en poids de polymère super absorbant.

8. Serviette hygiénique selon la revendication 4 ou l'une quelconque des revendications dépendantes de celle-ci, dans laquelle ladite couche absorbante est une seconde couche absorbante (48), et ledit système absorbant comportant en outre une première couche absorbante (46) au-dessus de ladite seconde couche absorbante (48).

9. Serviette hygiénique selon la revendication 8, dans laquelle ladite première couche absorbante est formée par air-laid sur la strate supérieure de pulpe de ladite seconde couche.

10. Serviette hygiénique selon l'une quelconque des revendications 1 à 9, dans laquelle ladite serviette hygiénique comporte une fixation (58) pour retenir ladite serviette hygiénique sur un sous-vêtement d'une utilisatrice.

11. Serviette hygiénique selon la revendication 8 ou l'une quelconque des revendications dépendantes de celle-ci, dans laquelle ladite seconde couche absorbante a un grammage dans la plage de 150 g/m² à 350 g/m², de manière préférée de 200 g/m² à 300 g/m², de manière plus préférée d'environ 250 g/m².

12. Serviette hygiénique selon la revendication 5 ou l'une quelconque des revendications dépendantes de celle-ci, dans laquelle la strate médiane comporte une première strate médiane adjacente à la strate inférieure, et une seconde strate médiane adjacente à la strate supérieure.
